# EUROPEAN PATENT SPECIFICATION

(11) **EP 4 493 131 B1**
(45) Date of publication and mention of the grant of the patent: **02.09.2026**
(21) Application number: 23709052.7
(22) Date of filing: 01.02.2023
(51) Int. Cl.: A61F 5/44, A61F 5/445

(54) **OSTOMY POUCH WITH VIEWING OPTION WINDOW**
OSTOMIEBEUTEL MIT SICHTFENSTER
POCHE DE STOMIE AVEC FENÊTRE D'OPTION DE VISUALISATION

(30) Priority: 17.03.2022 US 202263320804 P
(43) Date of publication of application: 22.01.2025
(73) Proprietor: Hollister Incorporated, Libertyville, IL 60048 (US)
(72) Inventor: WEINBERG, Robert J., Libertyville, IL 60048 (US)
(74) Representative: FRKelly
(86) International application number: PCT/US2023/061761
(87) International publication number: WO 2023/177940

(56) References cited:
- WO-A1-2021/165705
- US-A1- 2008 269 700
- US-A1- 2011 028 923
- US-A1- 2021 369 493

## Description

### BACKGROUND

The present disclosure pertains to an ostomy pouch. More particularly, the present disclosure pertains to an ostomy pouch with a viewing option window.

Ostomy pouches for collecting body waste are used by patients who have had surgery such as a colostomy, ileostomy, or urostomy. Ostomy pouches typically include flat, opposing side walls secured together along their edges to define a collection cavity. One of the side walls is provided with an opening to receive a stoma, and means to secure the pouch to the user, such as an adhesive barrier, so that body waste discharged through the stoma is received within the cavity.

In order to determine whether an ostomy bag is installed properly or to inspect a stoma, a user or clinician may wish to view the stoma while the ostomy pouch is secured to the user. Similarly, the body waste discharge may also need to be inspected while the ostomy pouch is secured to the user. Thus, it is desirable to provide an ostomy pouch system including an option to view the stoma and interior of the pouch, while still covering the pouch wall for user's discretion.

Accordingly, there is a need for an improved ostomy pouch that allows a view of the stoma or stoma output.

US2021369493A1 relates to an ostomy pouch 10 and discloses the features defined in the preamble of claim 1. The pouch 10 includes inner and outer walls 18b, 20b of flexible sheet material which sealed about a periphery. The walls 18b, 20b define a cavity for containing a stomal output and a drain 32 for draining stomal output from the cavity. An opaque comfort layer 18a, 20a is provided, at over an outer surface of the inner and/or outer walls 18b, 20b. The drain 32 is moveable between a deployed position for draining the stomal output from the cavity and a stowed position for storage of the drain 32, with at least part of the drain 32 overlying a portion of the comfort layer 18a, 20a in the stowed position. The drain 32 is substantially opaque on one side, and at least partly transparent on an opposing side.

WO2021165705A1 discloses an ostomy pouch (1) or wound bag comprising a fluid tight collecting bag (2) for containing bodily fluid or waste matter. The collecting bag includes an opening (16) for receiving bodily fluid or waste matter. A flexible material cover (20) is provided that at least partially covers the collecting bag. The cover comprises a front part (26) arranged on a first side of the collecting bag that in use faces forward away from the wearer. The front part of the cover includes a first panel section (28) having a viewing window through which the opening is visible and a cover panel section (30) that is reconfigurable between a closed configuration in which at least part of the cover panel section covers and obscures the viewing window and the opening is obscured, and an open configuration in which the viewing window is uncovered and the stoma opening is at least partially visible.

US2008269700A1 discloses an ostomy pouch formed to conceal, obscure, hide, cloak, or otherwise prevent the viewing of contents of the ostomy pouch except for a "sight glass", "bullseye", area or window formed in the pouch and positioned therein for viewing at least a portion of a stoma receptor of the ostomy pouch. The ostomy pouch is formed by a plastic bag incorporating a stoma receptor or opening in one side of the bag and a transparent, translucent or clear window or area in another side of the bag. The transparent window is preferably, but not necessarily, positioned opposite and adjacent the stoma receptor. The ostomy pouch may be entirely sealed except for a stoma receptor orifice or may be open at one end for emptying its contents and incorporate or use a closure at the open end.

### BRIEF SUMMARY OF THE INVENTION

The invention is set out in the appended set of claims. The present invention provides an ostomy pouch as detailed in claim 1. Advantageous features are provided in dependent claims.

The foregoing general description and the following detailed description are examples only and are not restrictive of the present disclosure.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is an exploded-view drawing of an ostomy pouch according to an embodiment.
FIG. 2 is a front view of the ostomy pouch of FIG. 1 with a curtain covering an opening of a viewing window member.
FIG. 3 is a front view of the ostomy pouch of FIG. 2 without the curtain.
FIG. 4 is a front view of an ostomy pouch according to another embodiment.
FIG. 5 is a front view of an ostomy pouch according to another embodiment.

### DETAILED DESCRIPTION

While the present disclosure is susceptible of embodiment in various forms, there is shown in the drawings and will hereinafter be described presently preferred embodiments with the understanding that the present disclosure is to be considered an exemplification and is not intended to limit the disclosure to the specific embodiments illustrated. The words "a" or "an" are to be taken to include both the singular and the plural. Conversely, any reference to plural items shall, where appropriate, include the singular.

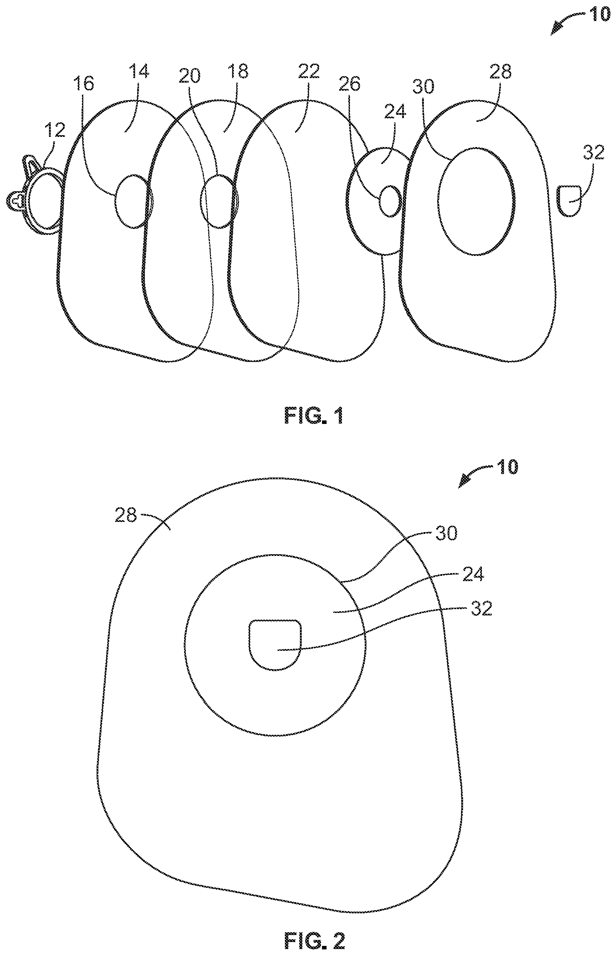

Referring now to the figures, FIGS. 1-3 show an ostomy pouch 10 including a viewing option according to an embodiment. The ostomy pouch 10 may generally include a patient attachment device 12, a body side comfort panel 14, a body side wall 18, a distal wall 22, a viewing window member 24, a distal comfort panel 28, and a curtain 32.

FIG. 1 shows an exploded view of the ostomy pouch 10. The body side wall 18 and the distal wall 22 may be sealed along their peripheral edges to define a cavity therein for collecting stoma output. The body side comfort panel 14 including a first opening 16 may be attached to the body side wall 18. The body side wall 18 may include a second opening 20, wherein the first and second openings 16, 20 may be aligned and configured to receive a stoma. The distal comfort panel 28 may be attached to the distal wall 22.

In this embodiment, the ostomy pouch 10 may be configured as a pouch for a 2-piece ostomy pouch system and the patient attachment device 12 may be a flange that includes a pouch side coupling member. The pouch side coupling member may be configured to engage with a barrier side coupling member (not shown) for securing the ostomy pouch 10 to a skin barrier (not shown). In use, the skin barrier may be attached to a user around a stoma and the ostomy pouch 10 may be attached to the skin barrier by engaging the coupling members, such that the patient attachment device12 may surround the stoma and stoma output may be received through the first and second openings 16, 18. In other embodiments, the ostomy pouch 10 may be configured as a one-piece ostomy pouch system including a skin barrier attached to a body side of the pouch and the patient attachment device 12 may be a 1-piece pouch with an attached barrier.

The distal wall 22 may be formed from a transparent film to allow viewing of the stoma and body waste collected in the pouch. The distal wall 22 may be covered with the distal comfort panel 28 formed from a suitable opaque material, such as a nonwoven or fabric. The distal comfort panel 28 may include a third opening 30 for viewing.

In this embodiment, the ostomy pouch 10 may include the viewing window member 24, which may be arranged between the distal wall 22 and the distal comfort panel 28. The viewing window member 24 may be configured to cover at least a portion of the distal wall 22 exposed through the third opening 30 to provide discretion for a user. The viewing window member 24 may include a viewing opening 26. In the embodiment of FIG. 1, each of the third opening 30 and the viewing opening 26 may have a generally circular shape, wherein the viewing opening 26 may have a diameter smaller than a diameter of the third opening 30. In other embodiments, the third opening 30 and the viewing opening 26 may be configured to have various shapes, such as rectangular openings, oval openings, and the like, wherein the size of the third opening 30 is greater than the size of the viewing opening 26, such that the viewing opening 26 may be arranged within the third opening 30.

The viewing window member 24 may be configured to be stretched by a user to enlarge the viewing opening 26 for viewing and substantially recover its original configuration when released. In an embodiment, the viewing window member 24 may be formed from a suitable resilient material, such as an elastomeric material. For example, the viewing window member 24 may be formed from a thermoplastic elastomer. In such an embodiment, the viewing window member 24 may be configured such that the viewing opening 26 may be stretched in various directions for desired viewing of the stoma or stoma output and spring back to the original size and configuration of the viewing opening 26 when released. The viewing window member 24 may be heat sealed to the distal wall 22 and/or the distal comfort panel 28. In another embodiment, the viewing window member 24 may be attached to a distal side of the distal comfort panel 28.

In the embodiment of FIGS. 1-3, the ostomy pouch may include the curtain 32 configured to cover the viewing opening 26. The curtain 32 may be separately formed from a suitable opaque material and attached to the viewing window member 24 to cover the viewing opening 26, such that a user may open the curtain 32 to access the viewing opening 26. In an embodiment, a portion of the curtain 32 may be sealed to the viewing window member 24 above the viewing opening 26 and configured to be opened by lifting up an unattached portion of the viewing window member 24.

FIG. 2 shows a front view of the ostomy pouch 10, wherein the curtain 32 is in a closed position covering the viewing opening 26 to prevent viewing of the stoma and pouch content through the viewing opening 26. In the closed position, the distal surface of the distal wall 22 may be completed covered by the distal comfort panel 28, the viewing window member 24, and the curtain 32.

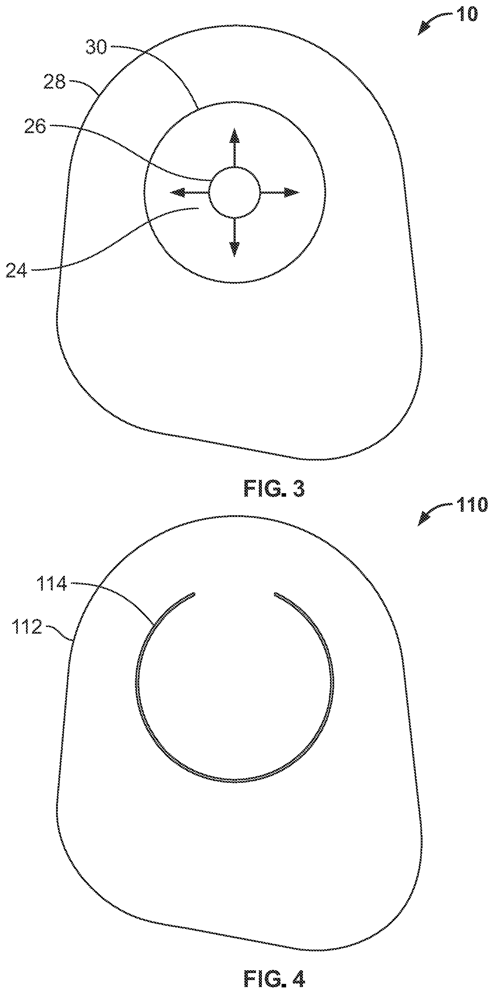

FIG. 3 shows a front view of ostomy pouch 10 without the curtain 32. The viewing window member 24 may be configured to allow a user to manually manipulated the viewing window member 24 to increase the size of the viewing opening 26 for viewing. For example, viewing window member 24 may be stretched in various directions, as shown by arrows on FIG. 3. The viewing window member 24 may be configured to constrict back to its original configuration when released.

FIG. 4 shows a front view of an ostomy pouch 110 according to another embodiment. The ostomy pouch 110 may be configured similar to ostomy pouch 10 of FIG.1 generally including a body side wall 18, a distal wall 22, a viewing window member 24, and a distal comfort panel 112, except in this embodiment, a curtain 114 may be integrally formed with the distal comfort panel 112. In this embodiment, the curtain 114 may be formed from a punched-out section of the distal comfort panel 112, wherein a portion of the curtain 114 remains attached to the distal comfort panel 112 while the other portion is unattached for opening of the curtain 114. The viewing window member 24 may be arranged under the curtain 114 and exposed when the curtain 114 is opened. For example, the curtain 114 may be lifted up, moved away from the distal comfort panel 112, such that a user may access the viewing window member 24 to stretch and enlarge the viewing opening 26 to view the stoma or stoma output.

In an embodiment, the ostomy pouch 110 may include a curtain securing member (not shown) configured to maintain the curtain 114 in a closed position to prevent the curtain 114 from opening accidentally. For example, the curtain securing member may include a first fastener provided on the viewing window member 24 and a second fastener provided on the curtain 114, wherein the first and second fasteners may be configured to engage with each other to hold the curtain in a closed position.

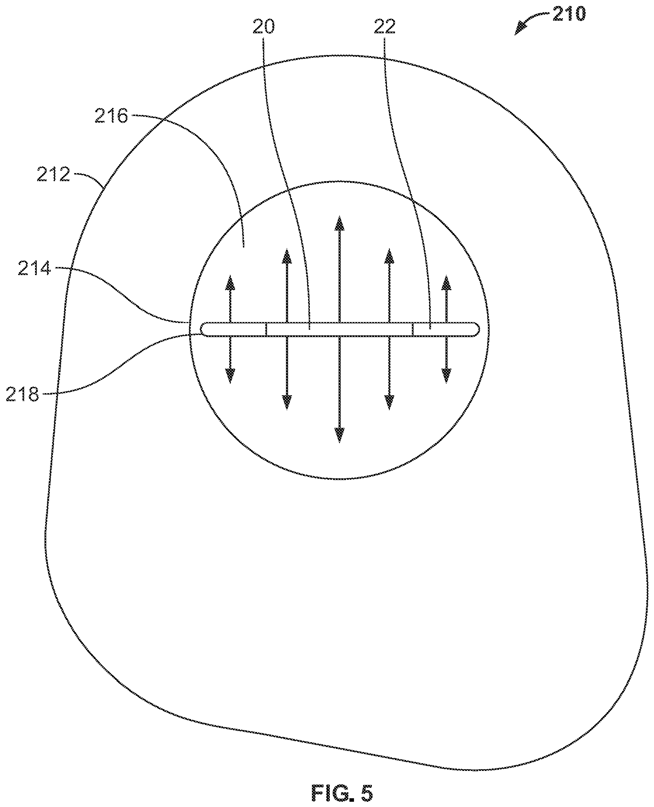

FIG. 5 shows a front view of an ostomy pouch 210 according to an embodiment. The ostomy pouch 210 may be configured similar to ostomy pouch 10 of FIG. 1 generally including a body side wall 18, a second opening 20, a distal wall 22, a viewing window member 216, and a distal comfort panel 212, except in this embodiment, the viewing opening 26 may be defined by a slit-like opening 218. The distal comfort panel 212 may include a third opening 214 for viewing.

In this embodiment, the slit-like opening 218 may be formed as a relatively thin opening that extends horizontally across the viewing window member 216. In another embodiment, the slit-like opening 218 may be formed as a horizontally extending cut in the viewing window member 216. In other embodiments, the slit-like opening 218 may be formed as a relatively thin opening that extends vertically across the viewing window member 216 or a vertically extending cut in the viewing window member 216. As it was with the viewing window member 24, the viewing window member 216 may be configured to be a resilient member which may be manipulated by a user to increase the size of the viewing opening 218 and recover its original shape and size when released. In an embodiment, the viewing window member 216 may be stretched in a direction perpendicular to the slit-like opening 218 to separate the viewing window member 216 at the slit-like opening 218 for viewing. For example, the slit-like opening 218 may be stretched to open in the directions shown by arrows in FIG. 5.

In an embodiment, ostomy pouch 210 may include a curtain (not shown) configured to cover the slit-like opening 218. The curtain may be attached to the distal comfort panel 212. For example, the curtain may be configured sufficiently wide to cover the entire slit-like opening 218.

From the foregoing it will be observed that numerous modifications and variations can be effectuated without departing from the true scope of the novel concepts of the present disclosure. It is to be understood that no limitation with respect to the specific embodiments illustrated is intended or should be inferred. The disclosure is intended to cover by the appended claims all such modifications as fall within the scope of the claims.

## Claims

1. An ostomy pouch (10, 110, 210) comprising:
a body side wall (18) and a distal wall (22) joined along their peripheral edges to define a cavity therebetween for collecting body waste;
an inlet opening defined in the body side wall configured to receive a stoma; and
a viewing window member (24) formed from an elastomeric material covering at least a portion of the distal wall, wherein the viewing window member includes a viewing opening (26) having a first configuration, and wherein the viewing window member is configured to be stretched to increase a size of the viewing opening (26) for viewing an interior of the ostomy pouch and recover the first configuration when released, wherein at least a portion of the distal wall (22) is formed from a transparent material; and
**characterised in** comprising a distal comfort panel (28, 112, 212) attached to and covering the distal wall, wherein the distal comfort panel is formed from an opaque material and includes a first opening (30), and wherein the viewing window member is arranged such that the viewing opening is arranged within the first opening.

2. The ostomy pouch (10, 110, 210) of claim 1, wherein the viewing window member (24) is formed from an opaque elastomeric material.

3. The ostomy pouch (10, 110, 210) of any of claims 1 -2, wherein the viewing window member (24) is formed from a thermoplastic elastomer.

4. The ostomy pouch (10, 110, 210) of claim 1, wherein the viewing opening (26) is a circular opening having a diameter smaller than a diameter of the first opening, wherein the viewing window member (24) is configured to be stretch in various directions to increase the size of the viewing opening and return to the first configuration when released.

5. The ostomy pouch (10, 110, 210) of claim 1, wherein the viewing opening (26) comprises a slit-like opening (218) extending across the viewing window member (24), wherein the viewing window member is configured to be stretched and separated at the slit-like opening to provide a viewing window and return to the first configuration when released.

6. The ostomy pouch (10, 110, 210) of claim 5, wherein the slit-like opening (218) is formed from a cut extending across at least a portion of the viewing window member (24).

7. The ostomy pouch (10, 110, 210) of any of claims 1-6, wherein the viewing window member (24) is arranged between the distal wall (22) and the distal comfort panel (28, 112, 212), wherein the viewing window member is sealed to the distal comfort panel.

8. The ostomy pouch (10, 110, 210) of any of claims 1-7, wherein the viewing window member (24) is arranged between the distal wall and the distal comfort panel, wherein the viewing window member is sealed to the distal wall.

9. The ostomy pouch (10, 110, 210) of any of claims 1-8, wherein the ostomy pouch includes a curtain (32, 114) formed from an opaque material and configured to cover the viewing opening.

10. The ostomy pouch (10, 110, 210) of claim 9, wherein the curtain (32, 114) includes a first portion attached to the viewing window member (24) and a second unattached portion, wherein the curtain is configured to be opened by lifting up the second unattached portion to provide an access to the viewing opening.

11. The ostomy pouch (10, 110, 210) of claim 9, wherein the curtain (32, 114) is integrally formed with the distal comfort panel (28, 112, 212).

12. The ostomy pouch (10, 110, 210) of claim 11, wherein the curtain (32, 114) is formed from a punched-out section of the distal comfort panel (28, 112, 212), wherein a first portion of the punched-out section remains connected to the comfort panel while a second portion of the punched-out section is unattached from the distal comfort panel, and wherein the curtain is configured to be opened by moving the second portion.

13. The ostomy pouch (10, 110, 210) of any of claims 9-12, wherein the ostomy pouch includes a curtain securing member configured to hold the curtain (32, 224) in a closed position.

## Patentansprüche

1. Ostomiebeutel (10, 110, 210), umfassend:
eine Körperseitenwand (18) und eine distale Wand (22), die entlang ihrer Umfangskanten verbunden sind, um dazwischen einen Hohlraum zum Sammeln von Körperausscheidungen zu definieren;
eine Einlassöffnung, die in der Körperseitenwand definiert und dazu konfiguriert ist, ein Stoma aufzunehmen; und
ein Sichtfensterelement (24), das aus einem elastomeren Material gebildet ist und mindestens einen Teil der distalen Wand bedeckt, wobei das Sichtfensterelement eine Sichtöffnung (26) mit einer ersten Konfiguration einschließt, und wobei das Sichtfensterelement dazu konfiguriert ist, gedehnt zu werden, um eine Größe der Sichtöffnung (26) zum Betrachten eines Inneren des Ostomiebeutels zu vergrößern und beim Loslassen die erste Konfiguration wiederherzustellen, wobei mindestens ein Teil der distalen Wand (22) aus einem transparenten Material gebildet ist; und
**dadurch gekennzeichnet**, umfassend eine distale Komfortplatte (28, 112, 212), die an der distalen Wand befestigt ist und diese bedeckt, wobei die distale Komfortplatte aus einem undurchsichtigen Material gebildet ist und eine erste Öffnung (30) einschließt, und wobei das Sichtfensterelement so angeordnet ist, dass die Sichtöffnung innerhalb der ersten Öffnung angeordnet ist.

2. Ostomiebeutel (10, 110, 210) nach Anspruch 1, wobei das Sichtfensterelement (24) aus einem undurchsichtigen elastomeren Material gebildet ist.

3. Ostomiebeutel (10, 110, 210) nach einem der Ansprüche 1-2, wobei das Sichtfensterelement (24) aus einem thermoplastischen Elastomer gebildet ist.

4. Ostomiebeutel (10, 110, 210) nach Anspruch 1, wobei die Sichtöffnung (26) eine kreisförmige Öffnung mit einem Durchmesser ist, der kleiner ist als ein Durchmesser der ersten Öffnung, wobei das Sichtfensterelement (24) dazu konfiguriert ist, in verschiedene Richtungen gedehnt zu werden, um die Größe der Sichtöffnung zu vergrößern und beim Loslassen in die erste Konfiguration zurückzukehren.

5. Ostomiebeutel (10, 110, 210) nach Anspruch 1, wobei die Sichtöffnung (26) eine schlitzartige Öffnung (218) umfasst, die sich über das Sichtfensterelement (24) erstreckt, wobei das Sichtfensterelement dazu konfiguriert ist, an der schlitzartigen Öffnung gedehnt und getrennt zu werden, um ein Sichtfenster bereitzustellen und beim Loslassen in die erste Konfiguration zurückzukehren.

6. Ostomiebeutel (10, 110, 210) nach Anspruch 5, wobei die schlitzartige Öffnung (218) durch einen Schnitt gebildet ist, der sich über mindestens einen Teil des Sichtfensterelements (24) erstreckt.

7. Ostomiebeutel (10, 110, 210) nach einem der Ansprüche 1-6, wobei das Sichtfensterelement (24) zwischen der distalen Wand (22) und der distalen Komfortplatte (28, 112, 212) angeordnet ist, wobei das Sichtfensterelement mit der distalen Komfortplatte versiegelt ist.

8. Ostomiebeutel (10, 110, 210) nach einem der Ansprüche 1-7, wobei das Sichtfensterelement (24) zwischen der distalen Wand und der distalen Komfortplatte angeordnet ist, wobei das Sichtfensterelement mit der distalen Wand versiegelt ist.

9. Ostomiebeutel (10, 110, 210) nach einem der Ansprüche 1-8, wobei der Ostomiebeutel einen Vorhang (32, 114) einschließt, der aus einem undurchsichtigen Material gebildet und dazu konfiguriert ist, die Sichtöffnung abzudecken.

10. Ostomiebeutel (10, 110, 210) nach Anspruch 9, wobei der Vorhang (32, 114) einen ersten Abschnitt, der an dem Sichtfensterelement (24) befestigt ist, und einen zweiten, nicht befestigten Abschnitt einschließt, wobei der Vorhang dazu konfiguriert ist, durch Anheben des zweiten, nicht befestigten Abschnitts geöffnet zu werden, um einen Zugang zur Sichtöffnung bereitzustellen.

11. Ostomiebeutel (10, 110, 210) nach Anspruch 9, wobei der Vorhang (32, 114) einstückig mit der distalen Komfortplatte (28, 112, 212) ausgebildet ist.

12. Ostomiebeutel (10, 110, 210) nach Anspruch 11, wobei der Vorhang (32, 114) aus einem ausgestanzten Abschnitt der distalen Komfortplatte (28, 112, 212) gebildet ist, wobei ein erster Abschnitt des ausgestanzten Abschnitts mit der Komfortplatte verbunden bleibt, während ein zweiter Abschnitt des ausgestanzten Abschnitts von der distalen Komfortplatte gelöst ist, und wobei der Vorhang dazu konfiguriert ist, durch Bewegen des zweiten Abschnitts geöffnet zu werden.

13. Ostomiebeutel (10, 110, 210) nach einem der Ansprüche 9-12, wobei der Ostomiebeutel ein Vorhangsicherungselement einschließt, das dazu konfiguriert ist, den Vorhang (32, 224) in einer geschlossenen Stellung zu halten.

## Revendications

1. Poche de stomie (10, 110, 210) comprenant :
une paroi latérale de corps (18) et une paroi distale (22) jointes le long de leurs bords périphériques pour définir une cavité entre elles pour collecter les déchets corporels ;
une ouverture d'entrée définie dans la paroi latérale de corps configurée pour recevoir une stomie ; et
un élément de fenêtre de visualisation (24) formé d'un matériau élastomère recouvrant au moins une partie de la paroi distale, dans lequel l'élément de fenêtre de visualisation comporte une ouverture de visualisation (26) ayant une première configuration, et dans lequel l'élément de fenêtre de visualisation est configuré pour être étiré pour augmenter une taille de l'ouverture de visualisation (26) pour visualiser un intérieur de la poche de stomie et pour reprendre la première configuration lorsqu'il est relâché, dans lequel au moins une partie de la paroi distale (22) est formée d'un matériau transparent ; et
**caractérisé en ce qu'**il comprend un panneau de confort distal (28, 112, 212) fixé à et recouvrant la paroi distale, dans lequel le panneau de confort distal est formé d'un matériau opaque et comporte une première ouverture (30), et dans lequel l'élément de fenêtre de visualisation est disposé de sorte que l'ouverture de visualisation est disposée à l'intérieur de la première ouverture.

2. Poche de stomie (10, 110, 210) selon la revendication 1, dans laquelle l'élément de fenêtre de visualisation (24) est formé d'un matériau élastomère opaque.

3. Poche de stomie (10, 110, 210) selon l'une quelconque des revendications 1 à 2, dans laquelle l'élément de fenêtre de visualisation (24) est formé d'un élastomère thermoplastique.

4. Poche de stomie (10, 110, 210) selon la revendication 1, dans laquelle l'ouverture de visualisation (26) est une ouverture circulaire ayant un diamètre inférieur qu'un diamètre de la première ouverture, dans laquelle l'élément de fenêtre de visualisation (24) est configuré pour être étiré dans différentes directions pour augmenter la taille de l'ouverture de visualisation et revenir à la première configuration lorsqu'il est relâché.

5. Poche de stomie (10, 110, 210) selon la revendication 1, dans laquelle l'ouverture de visualisation (26) comprend une ouverture de type fente (218) s'étendant à travers l'élément de fenêtre de visualisation (24), dans laquelle l'élément de fenêtre de visualisation est configuré pour être étiré et séparé au niveau de l'ouverture de type fente pour fournir une fenêtre de visualisation et revenir à la première configuration lorsqu'il est relâché.

6. Poche de stomie (10, 110, 210) selon la revendication 5, dans laquelle l'ouverture de type fente (218) est formée à partir d'une découpe s'étendant à travers au moins une partie de l'élément de fenêtre de visualisation (24).

7. Poche de stomie (10, 110, 210) selon l'une quelconque des revendications 1 à 6, dans laquelle l'élément de fenêtre de visualisation (24) est disposé entre la paroi distale (22) et le panneau de confort distal (28, 112, 212), dans laquelle l'élément de fenêtre de visualisation est scellé au panneau de confort distal.

8. Poche de stomie (10, 110, 210) selon l'une quelconque des revendications 1 à 7, dans laquelle l'élément de fenêtre de visualisation (24) est disposé entre la paroi distale et le panneau de confort distal, dans laquelle l'élément de fenêtre de visualisation est scellé à la paroi distale.

9. Poche de stomie (10, 110, 210) selon l'une quelconque des revendications 1 à 8, dans laquelle la poche de stomie comporte un rideau (32, 114) formé d'un matériau opaque et configuré pour couvrir l'ouverture de visualisation.

10. Poche de stomie (10, 110, 210) selon la revendication 9, dans laquelle le rideau (32, 114) comporte une première partie fixée à l'élément de fenêtre de visualisation (24) et une seconde partie non fixée, dans laquelle le rideau est configuré pour être ouvert en soulevant la seconde partie non fixée afin de fournir un accès à l'ouverture de visualisation.

11. Poche de stomie (10, 110, 210) selon la revendication 9, dans laquelle le rideau (32, 114) fait partie intégrante du panneau de confort distal (28, 112, 212).

12. Poche de stomie (10, 110, 210) selon la revendication 11, dans laquelle le rideau (32, 114) est formé à partir d'une section découpée du panneau de confort distal (28, 112, 212), dans laquelle une première partie de la section découpée reste connectée au panneau de confort tandis qu'une seconde partie de la section découpée est détachée du panneau de confort distal, et dans laquelle le rideau est configuré pour être ouvert en déplaçant la seconde partie.

13. Poche de stomie (10, 110, 210) selon l'une quelconque des revendications 9 à 12, dans laquelle la poche de stomie comporte un élément de fixation de rideau configuré pour maintenir le rideau (32, 224) en position fermée.
